# EUROPÄISCHE PATENTSCHRIFT

(11) **EP 2 000 805 B1**
(45) Veröffentlichungstag und Bekanntmachung des Hinweises auf die Patenterteilung: **17.07.2013**
(21) Anmeldenummer: 08008080.7
(22) Anmeldetag: 26.04.2008
(51) Int. Cl.: C07K 14/75, G01N 33/96, A61K 35/16, G01N 33/86

(54) **Stabile D-Dimer Flüssigzubereitung**
Stable D-dimer fluid preparation
Préparation liquide D-Dimer stable

(30) Priorität: 04.06.2007 DE 102007026153
(43) Veröffentlichungstag der Anmeldung: 10.12.2008
(73) Patentinhaber: Siemens Healthcare Diagnostics Products GmbH, 35041 Marburg (DE)
(72) Erfinder: Pilgrim, Sabine, Dr., 35037 Marburg (DE); Zander, Norbert, Dr., 35039 Marburg (DE)

(56) Entgegenhaltungen:
- EP-A- 1 695 984
- WO-A-02/39114
- WO-A-2007/089665
- DEMPFLE C-E: "D-dimer testing and venous thromboembolism: four view points." JOURNAL OF THROMBOSIS AND HAEMOSTASIS : JTH FEB 2005, Bd. 3, Nr. 2, Februar 2005 (2005-02), Seiten 377-379, XP002490512 ISSN: 1538-7933
- EDLUND ET AL: "A proposed stoichiometrical calibration procedure to achieve transferability of D-dimer measurements and to characterize the performance of different methods" CLINICAL BIOCHEMISTRY, PERGAMON PRESS, XX, Bd. 39, Nr. 2, 1. Februar 2006 (2006-02-01), Seiten 137-142, XP005299994 ISSN: 0009-9120
- Wagner C, and Dati, F.: "Fibrinogen" In: Thomas, L.: "Clinical Laboratory Diagnostics", 1 January 1998 (1998-01-01), TH-Books Verlagsgesellschaft mbH, Frankfurt/Main, Germany pages 609-610,

## Beschreibung

Die Erfindung liegt auf dem Gebiet der Gerinnungsdiagnostik und betrifft eine flüssige Zubereitung bestehend aus einer D-Dimer-haltigen Puffermatrix, die sich als Standardmaterial zu Kontroll- oder Kalibrierungszwecken eignet.

D-Dimer ist ein bedeutender Laborparameter zum Nachweis der Gerinnungsaktivierung. Das Ziel der Blutgerinnung ist die Bildung eines Blutgerinnsels (Thrombus), das im Verletzungsfall durch den Verschluss der Wunde den Blutverlust so gering wie möglich halten soll. Die Umwandlung von ubiquitär im Blut vorkommenden Fibrinogen in unlösliches Fibrin ist ein wesentlicher Bestandteil der Blutgerinnselbildung. Die Fibrin-Vorstufe Fibrinogen ist ein großmolekulares, dimeres Glykoprotein, welches aus drei Kettenpaaren besteht (α-, β- und γ-Ketten), die durch Disulfidbrücken miteinander verbunden sind. Die Fibrinbildung erfolgt durch eine Folge enzymatischer, proteolytischer Prozesse. Zunächst spaltet Thrombin das Fibrinopeptid A von den N-terminalen Regionen der α-Ketten und dann das Fibrinopeptid B von den β-Ketten des Fibrinogens, wodurch das DesAABB-Fibrin in Monomerform entsteht. Diese löslichen Fibrinmonomere polymerisieren spontan zu langen Fasern und schließlich zu einem dichten verzweigten Netz, wobei diese Form des Fibrins noch löslich ist. Dieses noch relativ instabile Fibrin wird schließlich durch die Aktivität des Blutgerinnungsfaktor Xllla (F Xllla) stabilisiert, indem F Xllla zunächst die Y-Ketten des Fibrins zu Dimeren quervernetzt und schließlich die α-Ketten zu Polymeren. Als Gegenspieler des Blutgerinnungssystems agiert das Fibrinolysesystem, dessen Aufgabe es ist, Fibringerinnsel wieder aufzulösen und damit z. B. die Rekanalisation des Blutgefäßes zu gewährleisten. Während der Fibrinolyse werden sowohl Fibrinogen als auch Fibrin durch Plasmin proteolytisch gespalten, wobei verschiedene, nun wieder wasserlösliche Spaltprodukte entstehen. Die Degradationsprodukte von Fibrinogen und Fibrin unterscheiden sich. Das kleinste Fibrin-spezifische Degradationsprodukt ist das D-Dimer, das aus durch FXllla kovalent verbundenen D-Domänen besteht. D-Dimer eignet sich daher als Marker der Fibrinolyse und stellt einen bedeutenden diagnostischen Parameter zum Nachweis der Gerinnungsaktivierung dar. Antikörper, die spezifisch D-Dimere erkennen, werden daher in der Diagnostik der intravasalen Gerinnungsaktivierung eingesetzt. Die quantitative Bestimmung des D-Dimer-Antigens in Plasma- oder Serumproben gibt Informationen zum Ausschluss venöser Thrombosen und Lungenembolie und wird zur Diagnose der disseminierten intravasalen Gerinnung herangezogen.

Im Stand der Technik sind verschiedene Testverfahren zum quantitativen Nachweis von D-Dimer in humanen Plasma- oder Serumproben bekannt. D-Dimer-spezifische Antikörper ermöglichen sowohl die Anwendung von immunchemischen Verfahren wie Latex-Agglutinationsassays oder von Enzym-Immunoassays. Bei den diversen D-Dimer Testverfahren ist es üblich, D-Dimerhaltige Präparationen unterschiedlicher D-Dimer Antigenkonzentration als Kontrollen oder Kalibratoren einzusetzen, wobei Kontrollen für den normalen Messbereich üblicherweise weniger als 0,5 mg/L FEU D-Dimer enthalten und Kontrollen für den pathologischen Messbereich mehr als 0,5 mg/L FEU D-Dimer. FEU bedeutet Fibrinogen äquivalente Einheit und ist die gängigste, weltweit benutzte Einheit für D-Dimer. 1 mg/L D-Dimer entsprechen dabei 2 mg/L FEU D-Dimer, wobei die Umrechnung darauf basiert, dass aus einem Fibrinogenmolekül nach Fibrinbildung und anschließender Fibrinolyse zwei D-Dimer Moleküle entstehen.

Die D-Dimer Kontrollen oder Kalibratoren für die Qualitätskontrolle kommerzieller D-Dimer Teste bestehen üblicherweise aus einer Plasmamatrix und werden im wesentlichen aus stabilisiertem, humanen Plasma hergestellt, welchem definierte Mengen an D-Dimer zugegeben werden. D-Dimer kann bekanntermaßen z. B. dadurch gewonnen werden, dass humanes Plasma mit einem Gerinnungsaktivator und anschließend mit Plasmin behandelt wird, wodurch zunächst die Fibrinbildung und anschließend die Fibrindegradation ausgelöst wird. Die in diesem sog. Clot-Lyse-Plasma durch die Fibrindegradation entstandenen D-Dimere können angereichert und gereinigt werden. Alternativ kann zur Gewinnung von D-Dimer auch reines Fibrinogen durch Zugabe von Calciumchlorid, F Xllla und Thrombin zu Fibrin polymerisiert werden, welches anschließend durch Inkubation mit Plasmin degradiert werden kann.

Der Großteil dieser bekannten Kontrollen oder Kalibratoren wird derzeit aus Stabilitätsgründen nach der Abfüllung lyophilisiert. Prinzipiell ist es aber auch möglich, flüssige, nicht lyophilisierte D-Dimer Kontrollen oder Kalibratoren mit hinreichender Haltbarkeit herzustellen. Bio-Rad Laboratories bietet z. B. flüssige D-Dimer Kontrollen auf humaner Plasmabasis an (Liquichek^{™} D-Dimer Control, Level 1, 2 und 3) deren Laufzeiten mit drei Jahren angegeben werden. Ein anderes flüssiges D-Dimer-haltiges Produkt, in dem D-Dimer in einer stabilisierten Puffermatrix vorlag, verfügte über eine Laufzeit von 18 Monaten. Die Stabilisierung erfolgte dabei über generelle, unspezifische Stabilisatoren wie Albumin vom Rind und Aprotinin als Inhibitor von Serinproteasen wie Thrombin oder Plasmin. Dempfle beschreibt eine Standardpräparation für D-Dimer-Teste, die eine physiologische Fibrinogenkonzentration aufweisen soll (Dempfle, C.-E-, D-dimer testing and venous thromboembolism: four view points. J Thromb Haemost 2005; 3: 377-9). D-Dimer-spezifische Stabilisatoren oder Prozeduren, welche zur Stabilisierung des Analyten in flüssiger Matrix besonders geeignet sind, sind jedoch nicht bekannt.

Die der Erfindung zugrunde liegende Aufgabe bestand also darin, eine Zubereitung bereitzustellen, die D-Dimer enthält und die im flüssigen Zustand über eine hinreichende Haltbarkeit verfügt, d. h. eine konstante Wiederfindung des Analyten D-Dimers gewährleistet.

Die Aufgabe wird durch die Bereitstellung einer Zubereitung gemäß Anspruch 1 gelöst.

Gegenstand der Erfindung ist eine Zubereitung bestehend aus einer Puffermatrix, die D-Dimer und zusätzlich Fibrinogen in einer Endkonzentration von 0.1 g/L bis 1 g/L, besonders bevorzugt von 0,2 g/L bis 0,5 g/L enthält. Es wurde gefunden, dass durch den Zusatz von Fibrinogen D-Dimer Zubereitungen erhalten werden, die bei einer Lagerungstemperatur von 37 °C für bis zu 24 Wochen eine hinreichend konstante Wiederfindung von D-Dimer gewährleisten, während D-Dimer Zubereitungen, denen kein Fibrinogen zugesetzt wird, bei einer Lagerungstemperatur von 37°C bereits nach 10 Wochen keine akzeptable Wiederfindung von D-Dimer mehr ermöglichen.

Die erfindungsgemäße Zubereitung besteht aus einer Puffermatrix und ist damit von Zubereitungen, die im wesentlichen aus einer Plasmamatrix bestehen, zu unterscheiden. Auf einer Plasmamatrix beruhende Zubereitungen, die als Kalibratoren oder Kontrollen verwendet werden, bestehen üblicherweise aus einem Normal- oder Mangelplasma und enthalten demnach endogenes Fibrinogen. Eine erfindungsgemäße Zubereitung basierend auf einer Puffermatrix besteht im wesentlichen aus einer gepufferten Lösung, die kein endogenes Fibrinogen enthält und welcher der Analyt D-Dimer sowie das Fibrinogen in gereinigter oder teilweise gereinigter Form zugegeben werden. Zur Herstellung der Puffermatrix sind vor allem Substanzen geeignet, die einen puffernden Effekt im pH-Wert-Bereich von 5 bis 10 aufweisen. Geeignet sind z. B. Phosphatpuffer, Acetatpuffer, Citratpuffer, Argininpuffer, Histidinpuffer und TRIS-Puffer. Besonders bevorzugt ist eine Puffermatrix mit einem pH-Wert von etwa 6 bis 9.

Die erfindungsgemäße Zubereitung bestehend aus einer Puffermatrix enthält D-Dimer in gewünschter Reinheit und Konzentration. Bevorzugterweise handelt es sich bei dem in der Zubereitung enthaltenen D-Dimer um D-Dimer aus humanem Clot-Lyse-Plasma oder um ein Degradationsprodukt von reinem, bevorzugt humanem Fibrinogen, das beispielsweise durch Zugabe von Calciumchlorid, F Xllla und Thrombin zu Fibrin polymerisiert wurde und anschließend durch Inkubation mit Plasmin, Urokinase oder Elastase oder einer Kombination davon degradiert wurde. Die Reinigung oder Anreicherung von D-Dimer kann durch ein beliebiges, dem Fachmann als geeignet erscheinendes Verfahren erfolgt sein. Je nach gewünschter Reinheit des D-Dimer können Reinigungsverfahren angewandt werden, die die Abtrennung von Begleitstoffen wie Kohlenhydraten, Lipiden, Nukleinsäuren, Proteinen und/oder anderen Biomolekülen ermöglichen. Beispiele für Verfahren, die bekanntermaßen zur Reinigung von Proteinen verwendet werden, sind chromatographische Trennverfahren, wie die lonenaustausch-, die Gelfiltrations-, die hydrophobe Interaktions- oder die Affinitätschromatographie. Daneben können auch die präparative Gelelektrophorese, die präparative isoelektrische Fokussierung, die Chromatofokussierung, die Fällung und die Ultrazentrifugation für die Reinigung von Proteinen aus einem Proteingemisch verwendet werden. Bevorzugterweise enthält eine erfindungsgemäße Zubereitung D-Dimer in einer Endkonzentration von etwa 0,1 bis etwa 100 mg/L FEU D-Dimer.

Die Zugabe von D-Dimer zu der Puffermatrix kann zum Beispiel auch dadurch erfolgen, dass ein D-Dimer-haltiges Clot-Lyse-Plasma ohne weitere Reinigung oder Anreicherung von D-Dimer mit einer wässrigen, gepufferten Lösung vermischt wird. Clot-Lyse-Plasma sollte aufgrund der Behandlung des Ausgangsplasmas mit einem Überschuss an Gerinnungsaktivator und der damit erzielten Fibrinbildung kein endogenes Fibrinogen mehr enthalten. In der Praxis ist jedoch nicht auszuschließen, dass Spuren von endogenem Fibrinogen, die -sofern sie überhaupt nachweisbar sind- keinen ausschlaggebenden Einfluss auf die Stabilität einer erfindungsgemäßen Zubereitung haben, in einem Clot-Lyse-Plasma enthalten sein können. Bevorzugterweise wird vor dem Vermischen die D-Dimer-Konzentration des Clot-Lyse-Plasmas bestimmt, und das Clot-Lyse-Plasma wird mit der wässrigen, gepufferten Lösung derart verdünnt, dass in der resultierenden Puffermatrix die gewünschte D-Dimer-Konzentration erreicht wird. Die resultierende Puffermatrix soll jedoch nicht mehr als 10 Volumenprozent Clot-Lyse-Plasma enthalten. Gegenstand der Erfindung sind demnach Zubereitungen, die aus einer Puffermatrix bestehen, welche 0 bis höchstens 10 Volumenprozent Clot-Lyse-Plasma enthält. Zu bevorzugen sind erfindungsgemäße Zubereitungen, die aus einer Puffermatrix bestehen, die höchstens 5 Volumenprozent, besonders bevorzugt höchstens 1 Volumenprozent, ganz besonders bevorzugt höchstens 0,5 Volumenprozent enthält.

Weiterhin enthält die Puffermatrix, aus der eine erfindungsgemäße Zubereitung besteht, Fibrinogen humanen, tierischen (z. B. aus Rind, Pferd, Katze, Hund, Kaninchen) oder rekombinanten Ursprungs. Bevorzugterweise handelt es sich bei dem in der Puffermatrix enthaltenen Fibrinogen um gereinigtes Fibrinogen, das auch kommerziell erhältlich ist. Die Gewinnung reinen Fibrinogens kann durch ein beliebiges, dem Fachmann als geeignet erscheinendes Verfahren erfolgt sein, das die Reinigung des Fibrinogens aus dem organischen Rohmaterial ermöglicht, in welchem Fibrinogen natürlicherweise vorkommt oder gentechnisch erzeugt wurde. Je nach gewünschter Reinheit des Fibrinogens können Reinigungsverfahren angewandt werden, die die Abtrennung von Begleitstoffen wie Kohlenhydraten, Lipiden, Nukleinsäuren, Proteinen und/oder anderen Biomolekülen ermöglichen. Rohmaterialien für die Gewinnung von Fibrinogen können z. B. tierische oder humane Gewebe oder Körperflüssigkeiten (z. B. Blut, Plasma) sein, Überstände oder Lysate tierischer oder humaner Zellkulturen, oder Kulturen eukaryontischer Zellen oder von Mikroorganismen, wie Bakterien oder Pilzen, die rekombinantes Fibrinogen exprimieren. Beispiele für Verfahren, die bekanntermaßen zur Reinigung von Proteinen verwendet werden, sind chromatographische Trennverfahren, wie die lonenaustausch-, die Gelfiltrations-, die hydrophobe Interaktions- oder die Affinitätschromatographie. Daneben können auch die präparative Gelelektrophorese, die präparative isoelektrische Fokussierung, die Chromatofokussierung, die Fällung und die Ultrazentrifugation für die Reinigung von Proteinen aus einem Proteingemisch verwendet werden. Die Puffermatrix, aus der eine erfindungsgemäße Zubereitung besteht, enthält Fibrinogen in einer Endkonzentration von 0,1 g/L bis 1 g/L, besonders bevorzugt von 0,2 g/L bis 0,5 g/L.

Bevorzugterweise kann die Puffermatrix, aus der eine erfindungsgemäße Zubereitung besteht, zusätzliche Substanzen zur Stabilisierung enthalten, wie z. B. Antioxidanzien oder reduzierende Agenzien zur Verhinderung von oxidativem Abbau, Proteinase-Inhibitoren zur Verhinderung von proteolytischen Prozessen, besonders bevorzugt Aprotinin, chelatisierende Agenzien zum Ausschluss von Schwermetallionen oder Bakteriostatika und Fungizide zur Vermeidung von mikrobiellem Wachstum, besonders bevorzugt Natriumazid. Aktivitätsverluste durch physikalische Effekte wie Adsorption, Denaturierung durch Oberflächen, Hitzedenaturierung, Trocknung, wiederholtes Einfrieren und Auftauen können z. B. durch Zugabe von Glycerin, Kohlenhydraten, Aminosäuren, hydrophilen Polymeren oder inerten Proteinen, wie humanem Serumalbumin (HSA), bovinem Serumalbumin (BSA) oder Ovalbumin, reduziert werden. Weiterhin kann eine erfindungsgemäße Zubereitung auch sogenannte Volumenersatzmittel enthalten, wie z. B. Polygeline (Haemaccel®).

Auch wenn der besondere Vorteil einer erfindungsgemäßen Zubereitung darin besteht, dass sie in flüssiger Form besonders stabil ist, ist es aber auch möglich die Zubereitung zu Lagerungszwecken zu lyophilisieren.

Ein weiterer Gegenstand der vorliegenden Erfindung ist die Verwendung einer erfindungsgemäßen Zubereitung als Kontrolle oder Kalibrator in einem Testverfahren, insbesondere in einem immunchemischen Testverfahren zur quantitativen Bestimmung von D-Dimer in humanen Serum- oder Plasmaproben. Besonders bevorzugt wird eine erfindungsgemäße Zubereitung zur Kontrolle oder Kalibration eines D-Dimer Testverfahrens verwendet, bei dem das D-Dimer Antigen mittels des monoklonalen Antikörper 8D3 nachgewiesen wird [Antikörper 8D3 siehe: Holvoet, P. et al. (1989) Binding properties of monoclonal antibodies against human fragment D-Dimer of cross-linked fibrin to human plasma clots in an in vivo model in rabbits. Thrombosis and Haemostasis 61(2): 307-313]. Bevorzugterweise werden dazu mehrere Zubereitungen unterschiedlicher D-Dimer Konzentration bereit gestellt, um normale (< 0,5 mg/L FEU) und pathologisch hohe (> 0,5 mg/L FEU) D-Dimer Level zu unterscheiden.

Die vorliegende Erfindung betrifft weiterhin ein Verfahren zur Herstellung einer erfindungsgemäßen Zubereitung, die D-Dimer enthält. Bei dem erfindungsgemäßen Verfahren wird eine D-Dimer Präparation in flüssiger Puffermatrix, z. B. gereinigtes D-Dimer aus humanem Clot-Lyse-Plasma gelöst in einer Puffermatrix, mit einer Fibrinogenlösung vermischt. In einer besonders bevorzugten Ausführungsform wird die Zubereitung nach dem Vermischen für eine begrenzte Zeitspanne bei +30 bis +50 °C inkubiert. Besonders bevorzugt erfolgt die Inkubation bei +37 °C. Dieser Inkubationsschritt bewirkt eine Stabilisierung der D-Dimer-Flüssigzubereitung. Bevorzugterweise erfolgt diese Inkubation für eine Zeitspanne von 4 Stunden bis zu 7 Tagen, besonders bevorzugt für 12 Stunden bis zu 3 Tagen, bevor die Zubereitung dann bei der gewünschten Lagerungstemperatur (üblicherweise im Kühlschrank bei +2 bis +8 °C) mehrere Monate bis zur weiteren Verwendung aufbewahrt werden kann.

### Figurenbeschreibung

**Figur 1**
   Haltbarkeit von D-Dimer in flüssiger Puffermatrix ohne Fibrinogen bei unterschiedlichen Lagerungstemperaturen (max, min: Toleranzgrenzen).
**Figur 2**
   Haltbarkeit von D-Dimer in einer erfindungsgemäßen Zubereitung in flüssiger Puffermatrix mit 0,25 g/L Fibrinogen bei unterschiedlichen Lagerungstemperaturen (max, min: Toleranzgrenzen).
**Figur 3**
   Haltbarkeit von D-Dimer in einer erfindungsgemäßen Zubereitung in flüssiger Puffermatrix mit 0,25 g/L Fibrinogen ohne Präinkubation bei +37 °C für 1 Woche (von -1 bis 0), sondern Inkubation bei +2 °C bis +8 °C, bei anschließend unterschiedlichen Lagerungstemperaturen. Der Zeitpunkt -1 Woche entspricht dem Zeitpunkt der Herstellung und der Sollwertermittlung (Sollwert = gestrichelte Linie).
**Figur 4**
   Haltbarkeit von D-Dimer in einer erfindungsgemäßen Zubereitung in flüssiger Puffermatrix mit 0,25 g/L Fibrinogen mit Präinkubation bei +37 °C für 1 Woche (von -1 bis 0) bei anschließend unterschiedlichen Lagerungstemperaturen. Der Zeitpunkt -1 Woche entspricht dem Zeitpunkt der Herstellung und der Sollwertermittlung (Sollwert = gestrichelte Linie).

Die im Folgenden beschriebenen Beispiele dienen der exemplarischen Beleuchtung einzelner Aspekte dieser Erfindung und sind nicht als Einschränkung zu verstehen:

### Beispiele

### Beispiel 1: Herstellung einer erfindungsgemäßen D-Dimer Zubereitung im kleinen Maßstab

Zur Gewinnung von D-Dimer wurde aus humanem Normalplasma durch Zugabe von Thrombin und anschließender Zugabe von Urokinase Clot-Lysis-Plasma hergestellt. Das so hergestellte Clot-Lysis-Plasma enthielt 1193 mg/L FEU D-Dimer und diente als D-Dimer Präparation zur Herstellung folgender Zubereitungen auf Basis einer Puffermatrix:

| | |
|---|---|
| Formulierung 1: | 3 mg/L FEU D-Dimer |
| | 50 mM NaH₂PO₄ |
| | 10 g/L bovines Serumalbumin |
| | 0,1 % (v/v) Tween® 20 |
| | 80.000 KIU/L Aprotinin |
| | 0,04 % (w/v) Natriumazid |
| | pH 6,9 |
| | |
| Formulierung 2: | 3 mg/L FEU D-Dimer |
| | 50 mM NaH₂PO₄ |
| | 10 g/L bovines Serumalbumin |
| | 0,1 % (v/v) Tween® 20 |
| | 80.000 KIU/L Aprotinin |
| | 0,04 % (w/v) Natriumazid |
| | 0,25 g/L humanes, gerinnungsfähiges Fibrinogen |
| | (Sigma-Aldrich, Katalognr.: F4883) |
| | pH 6,9 |

Die beiden Formulierungen mit je einem Gesamtvolumen von 0,15 L unterschieden sich nur dadurch, dass die erfindungsgemäße Formulierung 2 zusätzlich 0,25 g/L humanes Fibrinogen enthielt.

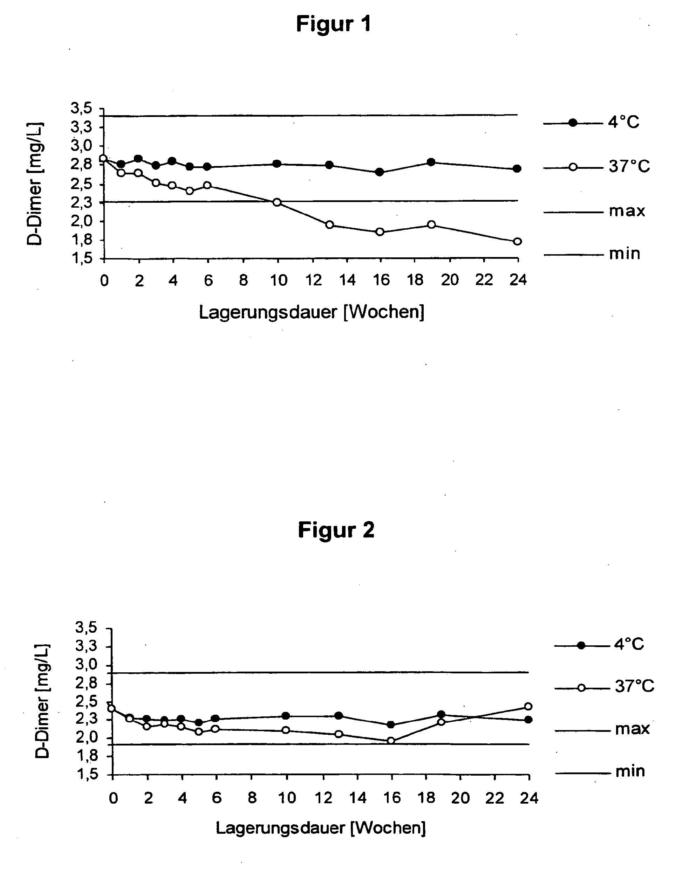

Beide Zubereitungen wurden nach dem Vermischen der Komponenten bei Raumtemperatur in Glasgefäße abgefüllt und für 3 Tage bei +37 °C inkubiert. Anschließend wurden die Zubereitungen bei +2 bis +8°C (gewünschte Lagerungstemperatur) bzw. bei +37°C (zur Generierung beschleunigter Haltbarkeitsdaten) gelagert. Nach entsprechenden Lagerungszeiträumen wurde die D-Dimer Konzentration mit einem Latexpartikel-verstärkten immunoturbidimetrischen Test, der auf dem Prinzip der Latexagglutination durch den D-Dimer spezifischen Antikörper 8D3 beruht (Innovance® D-Dimer Test, Dade Behring Marburg GmbH, Marburg, Deutschland), auf einem Gerinnungsmessgerät (BCS® System, Dade Behring Marburg GmbH, Marburg, Deutschland) bestimmt. Die Ergebnisse sind in den Figuren 1 und 2 dargestellt (Fig. 1: D-Dimer in flüssiger Puffermatrix ohne Fibrinogen (Formulierung 1); Fig. 2: D-Dimer in flüssiger Puffermatrix mit Fibrinogen (Formulierung 2).

Die Haltbarkeitsstudien zeigen eindeutig, dass die Fibrinogen-haltige D-Dimer Zubereitung während einer Lagerungszeit von 24 Wochen bei +37 °C einen konstante Wiederfindung ihrer D-Dimer Konzentration aufweist, wenn man den zwischen den Toleranzgrenzen (max, min) eingezeichneten Toleranzbereich als Akzeptanzkriterium festlegt. Der jeweilige Toleranzbereich (+/- 20 %) wurde anhand der nach der Präinkubation wiedergefundenen D-Dimer-Konzentration zum Zeitpunkt 0 festgelegt. Im Gegensatz dazu fällt die D-Dimer Zubereitung ohne Fibrinogen (Fig. 1) nach 10 Wochen Lagerungszeit bei +37 °C aus dem Toleranzbereich für eine akzeptable Haltbarkeit heraus. Dies zeigt, dass Fibrinogen die Haltbarkeit von D-Dimer in Puffermatrix verlängert.

### Beispiel 2: Herstellung von D-Dimer Zubereitungen im großen Maßstab mit und ohne Präinkubation bei +37 °C

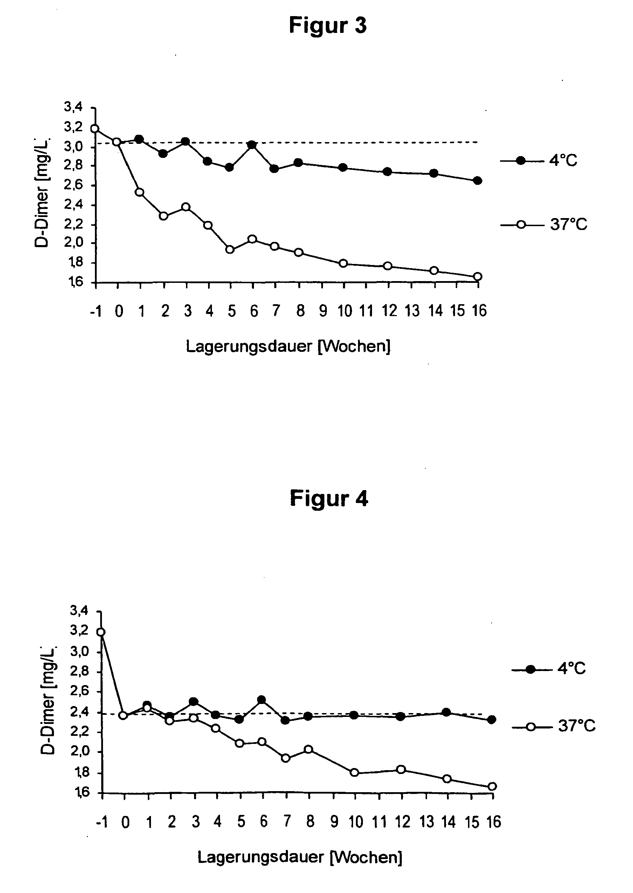

Um den Effekt einer Präinkubation der erfindungsgemäßen Zubereitung bei +37 °C zu untersuchen, wurden 10 L einer Fibrinogen-haltigen D-Dimer Zubereitung gemäß Formulierung 2 (siehe Beispiel 1) hergestellt. Ein Teil dieser Zubereitung wurde nach dem Vermischen der Komponenten bei Raumtemperatur für eine Woche bei +2 bis +8 °C gelagert. Parallel dazu wurde ein anderer Teil dieser Zubereitung für eine Woche bei +37 °C gelagert (präinkubiert). Anschließend wurde jeweils ein Teil der beiden Ansätze bei +2 bis +8 °C (gewünschte Lagerungstemperatur) bzw. bei +37°C (zur Generierung beschleunigter Haltbarkeitsdaten) gelagert. Nach entsprechenden Lagerungszeiträumen wurde die D-Dimer Konzentration bestimmt (siehe Beispiel 1). Die Ergebnisse sind in den Figuren 3 und 4 dargestellt.

Die Ergebnisse zeigen, dass nach Präinkubation der Fibrinogen-haltigen D-Dimer Zubereitung in Puffermatrix bei +37 °C die D-Dimer Konzentration zwar niedriger ist als zum Zeitpunkt der Herstellung, dass aber nach der Präinkubation die D-Dimer Konzentration nach einem Zeitraum von 16 Wochen bei +37 °C nur um 30 % und bei 4°C um 2 % im Vergleich zum Ausgangswert (0 Wochen) abfällt (Fig. 4). Für die gleiche Zubereitung, die jedoch nicht präinkubiert wurde, fiel die D-Dimer Konzentration nach einem Zeitraum von 16 Wochen bei +37 °C um 54 % und bei 4°C um 14 % ab (Fig. 3). Ein Präinkubationsschritt bei +37 °C verlängert demnach deutlich messbar die Haltbarkeit von Fibrinogen-haltigen D-Dimer Zubereitungen.

## Patentansprüche

1. Zubereitung bestehend aus einer Puffermatrix, die D-Dimer enthält, **dadurch gekennzeichnet, dass** die Puffermatrix zusätzlich Fibrinogen in einer Endkonzentration von 0,1 g/L bis 1 g/L, besonders bevorzugt von 0,2 g/L bis 0,5 g/L enthält.

2. Zubereitung nach Anspruch 1 **dadurch gekennzeichnet, dass** das Fibrinogen humanes oder tierisches Fibrinogen ist.

3. Zubereitung nach einem der vorhergehenden Ansprüche **dadurch gekennzeichnet, dass** die Zubereitung flüssig ist.

4. Zubereitung nach einem der vorhergehenden Ansprüche **dadurch gekennzeichnet, dass** die Puffermatrix etwa 0,1 bis etwa 100 mg/L FEU D-Dimer enthält.

5. Zubereitung nach einem der vorhergehenden Ansprüche **dadurch gekennzeichnet, dass** sie zusätzlich ein inertes Protein, bevorzugt Rinderserumalbumin, und/oder einen Proteaseinhibitor und/oder ein Detergenz und/oder einen Zusatz mit bakterizider und/oder fungizider Wirkung, bevorzugt Natriumazid, enthält.

6. Zubereitung nach einem der vorhergehenden Ansprüche **dadurch gekennzeichnet, dass** die Puffermatrix 0 bis höchstens 10 Volumenprozent Clot-Lyse-Plasma enthält.

7. Zubereitung nach Anspruch 6 **dadurch gekennzeichnet, dass** die Puffermatrix höchstens 5 Volumenprozent, bevorzugt höchstens 1 Volumenprozent, besonders bevorzugt höchstens 0,5 Volumenprozent Clot-Lyse-Plasma enthält.

8. Verwendung einer D-Dimer-enthaltenden Zubereitung gemäß einem der vorhergehenden Ansprüche zur Kontrolle oder Kalibration von Testverfahren zur Bestimmung von D-Dimer.

9. Verwendung gemäß Anspruch 8 zur Kontrolle oder Kalibration von Antikörperbasierten Testverfahren zur Bestimmung von D-Dimer, bei denen der monoklonale anti-D-Dimer Antikörper 8D3 verwendet wird.

10. Verfahren zur Herstellung einer Zubereitung gemäß Anspruch 1, die D-Dimer enthält, **dadurch gekennzeichnet, dass** eine D-Dimer-Lösung mit einer wässrigen, gepufferten Fibrinogen-Lösung vermischt wird.

11. Verfahren nach Anspruch 10 **dadurch gekennzeichnet, dass** die Zubereitung nach dem Vermischen für eine begrenzte Zeitspanne bei 30 bis 50 °C, bevorzugt bei 37 °C inkubiert wird.

12. Verfahren nach Anspruch 10 **dadurch gekennzeichnet, dass** die Zubereitung nach dem Vermischen für 4 Stunden bis 7 Tage, bevorzugt für 12 Stunden bis 3 Tage bei 30 bis 50 °C inkubiert wird.

13. Verwendung von Fibrinogen humanen oder tierischen Ursprungs zur Stabilisierung einer Zubereitung, die aus einer Puffermatrix besteht, die D-Dimer enthält.

## Claims

1. A preparation consisting of a buffer matrix which contains D-dimer, wherein the buffer matrix additionally contains fibrinogen in a final concentration of 0.1 g/l to 1 g/l, particularly preferably of 0.2 g/l to 0.5 g/l.

2. The preparation as claimed in claim 1, wherein the fibrinogen is human or animal fibrinogen.

3. The preparation as claimed in one of the preceding claims, wherein the preparation is liquid.

4. The preparation as claimed in one of the preceding claims, wherein the buffer matrix contains approximately 0.1 to approximately 100 mg/l of FEU D-dimer.

5. The preparation as claimed in one of the preceding claims, which additionally contains an inert protein, preferably bovine serum albumin, and/or a protease inhibitor and/or a detergent and/or an additive having bactericidal and/or fungicidal action, preferably sodium azide.

6. The preparation as claimed in one of the preceding claims, wherein the buffer matrix contains 0 to at most 10 percent by volume of clot lysis plasma.

7. The preparation as claimed in claim 6, wherein the buffer matrix contains at most 5 percent by volume, preferably at most 1 percent by volume, particularly preferably at most 0.5 percent by volume, of clot lysis plasma.

8. The use of a D-dimer-containing preparation as claimed in one of the preceding claims for the control or calibration of test procedures for the determination of D-dimer.

9. The use as claimed in claim 8 for the control or calibration of antibody-based test procedures for the determination of D-dimer, in which the monoclonal anti-D-dimer antibody 8D3 is used.

10. A procedure for the production of a preparation as claimed in claim 1 which contains D-dimer, wherein a D-dimer solution is mixed with an aqueous, buffered fibrinogen solution.

11. The procedure as claimed in claim 10, wherein the preparation is incubated for a limited period at 30 to 50°C, preferably at 37°C, after mixing.

12. The procedure as claimed in claim 10, wherein the preparation is incubated at 30 to 50°C for 4 hours to 7 days, preferably for 12 hours to 3 days, after mixing.

13. The use of fibrinogen of human or animal origin for the stabilization of a preparation which consists of a buffer matrix which contains D-dimer.

## Revendications

1. Préparation constituée d'une matrice tampon qui contient un dimère D, **caractérisée en ce que** la matrice tampon contient, en outre, du fibrinogène en une concentration finale de 0,1 g/L à 1 g/L, d'une manière particulièrement préférée de 0,2 g/L à 0,5 g/L.

2. Préparation suivant la revendication 1, **caractérisée en ce que** le fibrinogène est du fibrinogène humain ou animal.

3. Préparation suivant l'une des revendications précédentes, **caractérisée en ce que** la préparation est liquide.

4. Préparation suivant l'une des revendications précédentes, **caractérisée en ce que** la matrice tampon contient environ 0,1 à environ 100 mg/L de dimère D FEU.

5. Préparation suivant l'une des revendications précédentes, **caractérisée en ce qu'**elle comprend, en outre, une protéine inerte, de préférence de l'albumine sérique de boeuf et/ou un inhibiteur de protéase et/ou un détergent et/ou un additif ayant un effet bactéricide et/ou fongicide, de préférence de l'azide de sodium.

6. Préparation suivant l'une des revendications précédentes, **caractérisée en ce que** la matrice tampon contient de 0 à au plus 10 pourcent en volume de plasma de lyse de caillot.

7. Préparation suivant la revendication 6, **caractérisée en ce que** la matrice tampon contient au plus 5 pourcent en volume, de préférence au plus 1 pourcent en volume, d'une manière particulièrement préférée au plus 0,5 pourcent en volume, de plasma de lyse de caillot.

8. Utilisation d'une préparation contenant un dimère D suivant l'une des revendications précédentes pour le réglage ou l'étalonnage de procédés de test de détermination de dimère D.

9. Utilisation suivant la revendication 8 pour le réglage ou l'étalonnage de procédés de test à base d'anticorps pour la détermination de dimère D, dans laquelle on utilise l'anticorps monoclonal anti-dimère D 8D3.

10. Procédé de production d'une préparation suivant la revendication 1 qui contient un dimère D, **caractérisé en ce que** l'on mélange une solution de dimère D à une solution de fibrinogène aqueuse tamponnée.

11. Procédé suivant la revendication 10, **caractérisé en ce que** l'on fait incuber la préparation après le mélange pendant un laps de temps limité à 30 à 50°C, de préférence à 37°C.

12. Procédé suivant la revendication 10, **caractérisé en ce que** l'on fait incuber la préparation après le mélange pendant 4 heures à 7 jours, de préférence pendant 12 heures à 3 jours à 30 à 50°C.

13. Utilisation de fibrinogène d'origine humaine ou animale pour la stabilisation d'une préparation constituée d'une matrice tampon qui contient un dimère D.
